(19) Europäisches Patentamt
European Patent Office
Office européen des brevets

(11) **EP 0 892 619 B1**

(12) **EUROPÄISCHE PATENTSCHRIFT**

(45) Veröffentlichungstag und Bekanntmachung des Hinweises auf die Patenterteilung:
**05.01.2000 Patentblatt 2000/01**

(51) Int. Cl.[7]: **A61B 5/00**, G04B 47/00, G06F 3/02

(21) Anmeldenummer: **97920620.8**

(22) Anmeldetag: **07.04.1997**

(86) Internationale Anmeldenummer:
**PCT/EP97/01712**

(87) Internationale Veröffentlichungsnummer:
**WO 97/37585 (16.10.1997 Gazette 1997/44)**

(54) **GERÄT ZUR REGISTRIERUNG VON BEFINDLICHKEITSWERTEN**

EQUIPMENT FOR RECORDING A PATIENT'S STATE OF HEALTH

APPAREIL POUR L'ENREGISTREMENT DE VALEURS RELATIVES A UN ETAT DE SANTE

(84) Benannte Vertragsstaaten:
**AT BE CH DE DK ES FI FR GB GR IE IT LI LU MC NL PT SE**

(30) Priorität: **11.04.1996 DE 19614255**

(43) Veröffentlichungstag der Anmeldung:
**27.01.1999 Patentblatt 1999/04**

(73) Patentinhaber:
**Von Zitzewitz, Falk**
**70193 Stuttgart (DE)**

(72) Erfinder: **Von Zitzewitz, Falk**
**70193 Stuttgart (DE)**

(74) Vertreter:
**Heumann, Christian, Dipl.-Ing.**
**Patentanwalt,**
**Hospitalstrasse 8**
**70174 Stuttgart (DE)**

(56) Entgegenhaltungen:
**WO-A-94/06088**      **WO-A-96/25877**
**FR-A- 2 599 252**      **US-A- 4 975 842**

## Beschreibung

Die Erfindung betrifft ein tragbares Gerät zur Registrierung, Anzeige und Speicherung von subjektiven Befindlichkeitswerten nach dem Oberbegriff des Patentanspruches 1.

Es ist bekannt, bestimmte physiologische Werte eines Patienten durch am Körper tragbare Meßgeräte oder Fühler zu erfassen und in einem Registriergerät auf einem Datenträger zu speichern. Beispielsweise kann somit die Änderung des Blutdruckes eines Patienten über der Zeit gemessen und aufgezeichnet werden. Die Messung erfolgt dabei durch geeignete Meßgeräte, also automatisch ohne Einwirkung des Patienten. Ferner ist es bekannt, durch spezielle Meßgeräte die Handbewegungen eines Patienten hinsichtlich ihrer Amplitude zu erfassen und aufzuzeichnen, um dadurch ein verläßliches Bild vom Verlauf des Bewegungsverhaltens zu erhalten. Schließlich sind auch sogenannte Oxigo-Meter zur kontinuierlichen Messung des Sauerstoffgehaltes im Blut eines Patienten beim Atemnot- und Apnoe-Syndrom bekannt. Diese Geräte versagen, wenn es um die Erfassung von physikalisch nicht ohne weiteres meßbaren physiologischen Werten am Patienten geht. In solchen Fällen ist der Arzt auf die Befragung des Patienten nach seiner Befindlichkeit bzw. nach der Wirkung eines Medikaments angewiesen. Da die Beantwortung durch den Patienten aus der Erinnerung heraus erfolgt, ergeben sich oft Verfälschungen der Erlebnisse, Fehleinschätzungen und ungenaue Angaben, die daher für den Arzt nur von relativer Bedeutung sind, wenn nicht sogar unbrauchbar.

Durch die **DE-A 37 03 404** wurde bereits ein Taschen-Computer zur Registrierung von Patientendaten bekannt. Dabei gibt der Patient subjektive Befindlichkeitsparameter zu bestimmten Zeitpunkten, die durch ein Programm festgelegt sind, in das Gerät ein, welches die Daten mit der Uhrzeit und dem Datum speichert. Über Steuerknöpfe kann der Patient den Cursor auf einer Skala so einstellen, daß der Grad seiner subjektiven Beschwerde markiert und dann gespeichert wird. Dieses Gerät und die damit aufgenommenen Daten haben keinerlei Bezug zum Zeitpunkt der Medikamenteneinnahme durch den Patienten.

Duch die **DE-A 40 25 830** wurde ein batteriebetriebener Kleinrechner zur ambulanten Datendokumentation bekannt, bei welchem der Patient nach einem komplizierten Fragezyklus seine subjektiven Befindlichkeiten bewertet, mittels Tasten in das Gerät eingibt und speichert. Auch diese Datenabfrage und -eingabe hat nur einen mittelbaren Bezug zum Zeitpunkt der Medikamenteneinnahme. Darüber hinaus sind die eingegebenen Werte ohne Bezug zu dem vorherigen Wert, d.h. "absolut". Der Patient kann seinen zuletzt eingegebenen Wert nicht sehen, muß also eine absolute Neubewertung zu dem betreffenden Zeitpunkt vornehmen. Es ergibt sich somit keine zusammenhängende, aussagefähige Funktion des Befindlichkeitsverlaufs über der Zeit.

Durch die **WO 94/06088** wurde ein Verfahren bekannt, welches dem Patienten mittels eines tragbaren Computers Empfehlungen für die Einnahme von Medikamenten gibt. Der Patient selbst gibt verschiedene Daten, nach denen er vom Computer gefragt wird, in das Gerät ein, diese Daten werden verarbeitet, und zwar zu Anweisungen für die Verabreichung von Medikamenten bezüglich Dosis und Zeitpunkt, darüber hinaus wird ein Expertsystem konsultiert, welches letztendlich die für den Patienten verbindliche Empfehlung abgibt. Insofern stellt dieses Verfahren einen "Regelkreis" zwischen Patient und Computer dar, bei welchem der Computer offenbar die Rolle des Arztes einnimmt. Ein derartiges Verfahren und auch das für die Durchführung dieses Verfahrens notwendige Gerät sind sehr aufwendig und kompliziert und insofern gerade für Patienten, die in ihrer Wahrnehmung und Beweglichkeit eingeschränkt sind, nicht immer geeignet. Darüber hinaus müssen die Empfehlungen eines solchen "Computer-Arztes' selbstverständlich relativiert werden, da ein solches Gerät den wirklichen Arzt nicht ersetzen kann.

Schließlich wurde durch die **FR-A 2 599 252** ein tragbares Taschencomputergerät bekannt, welches als elektronischer, programmierbarer Wecker für die Medikamenteneinnahme ausgebildet ist. Der Patient wird durch ein akustisches Signal an die Einnahme von Medikamenten, die auf einem Display angezeigt werden, erinnert. Das Gerät selbst weist keine Eingabetasten auf, sondern lediglich Tasten zur Änderung der Anzeige am Display (sogenannte Rolltaste). Für die Programmierung des Weckers ist eine separate Tastatur vorgesehen. Das Gerät hat somit lediglich eine Weck- und Anzeigefunktion für den Patienten.

Aufgabe der Erfindung ist die Bereitstellung eines Gerätes zur Erfassung und Registrierung von objektiv nicht meßbaren physiologischen Werten bzw. Befindlichkeiten eines Patienten im Hinblick auf Medikamenteneinnahme, insbesondere eines in seiner Wahrnehmung und/oder motorischen Beweglichkeit eingeschränkten Patienten. Diese Aufgabe wird erfindungsgemäß durch die Merkmale der Ansprüche gelöst.

Durch das erfindungsgemäße Gerät ist es möglich, daß die subjektive Befindlichkeit des Patienten, insbesondere nach Medikamenteneinnahme, erfaßt und registriert wird, wobei dies durch den Patienten selbst erfolgt. Dadurch wird der Wirkungsverlauf, d.h. die Wirkungsänderung nach Medikamteneinnahme über der Zeit auf einem Datenträger aufgezeichnet und ist somit für den behandelnden Arzt sichtbar. Durch diese reproduzierbare Aufzeichnung ist eine Optimierung der Medikamenteneinstellung, insbesondere bei nur kurz wirksamen Medikamenten möglich. Das Ziel ist hierbei, eine gleichmäßigere Wirkung der Medikamente beim Patienten zu erreichen. Nebenwirkungen durch Überdosierung zu vermeiden und den Patienten durch zeitlich richtige Einnahme zu unterstützen, z.B. bei der Parkinsonschen Erkrankung. Darüber hinaus ergibt sich für den Patienten selbst der Vorteil, daß er durch die Registrierung der Wirkungsintensität und eventueller Nebenwirkungen nach Medikamenteneinnahme die Wirkung besser beurteilen kann. Ferner wird durch dieses Gerät die Bereitschaft des Patienten, konstruktiv mit dem Arzt zusammenzuarbeiten (sogenannte Compliance) gesteigert. Schließlich kann dieses Gerät auch vorteilhaft bei der Testung und Erprobung neuer Wirksubstanzen, Arzneimittel und Therapieverfahren (z.B. klinische Prüfung von Medikamenten, Naturheilverfahren, Bestrahlungen u.a.) angewendet werden, um den Wirkungsverlauf wesentlich genauer zu erfassen. Durch das erfindungsgemäße Gerät, d.h. durch den sehr bequem tragbaren, vom Patienten bedienbaren Apparat in der Größe einer Zigarettenschachtel oder Armbanduhr ist es auf einfache Weise möglich, die nach Medikamenteneinnahme auftretende subjektive Befindlichkeit, d.h. die subjektiv empfundene Wirkung des Medikamentes, das z.B. in Form einer Arzneimitteleinnahmeanweisung zu bestimmten Zeitpunkten, auch in unterschiedlichen Arzneimittelzusammensetzungen (hinsichtlich Wirkstoffmenge und -art) vorgegeben ist, und die Änderung der Wirkung über der Zeit durch Tasten in dieses Gerät einzugeben, dadurch aufzuzeichnen, zu registrieren und zu speichern. Am Ende einer solchen Aufzeichnung, die beliebig oft und durch einfachen Tastendruck wiederholt werden kann, sind die registrierten Werte als Funktion auf einem Datenträger gespeichert, der dem Gerät als Chip entnommen oder über einen Adapter direkt auf ein Analysegerät (z.B. einen PC) übertragen werden kann. Dieser Chip bzw. die Anzeige der Daten auf dem Bildschirm dient dem behandelnden Arzt bzw. dem Therapeuten als Kontrolle für die Medikamentenwirkung und dem Patienten gleichzeitig als Anweisung für die Medikamenteneinnahme.

Die Erfindung wird anschließend anhand verschiedener Ausführungsbeispiele beschrieben und ist darüber hinaus als Gerät in der Zeichnung dargestellt. Es zeigen

Fig. 1     eine vereinfachte Ausführung des tragbaren Registriergerätes,

Fig. 2     ein Diagramm der Funktion Wirkungsänderung über der Zeit des Gerätes gemäß Figur 1,

Fig. 3     eine erweiterte Ausführung des Registriergerätes in Form einer Armbanduhr mit optischen Anzeigefunktionen,

Fig. 4     ein Diagramm der gestuften Wirkungsfunktion des Gerätes gemäß Figur 3,

Fig. 5     eine weitere Ausführung des Registriergerätes mit erweiterten Eingabefunktionen und

Fig. 6     eine weitere Ausführung des Registriergerätes mit externem Taster und externem Meßfühler.

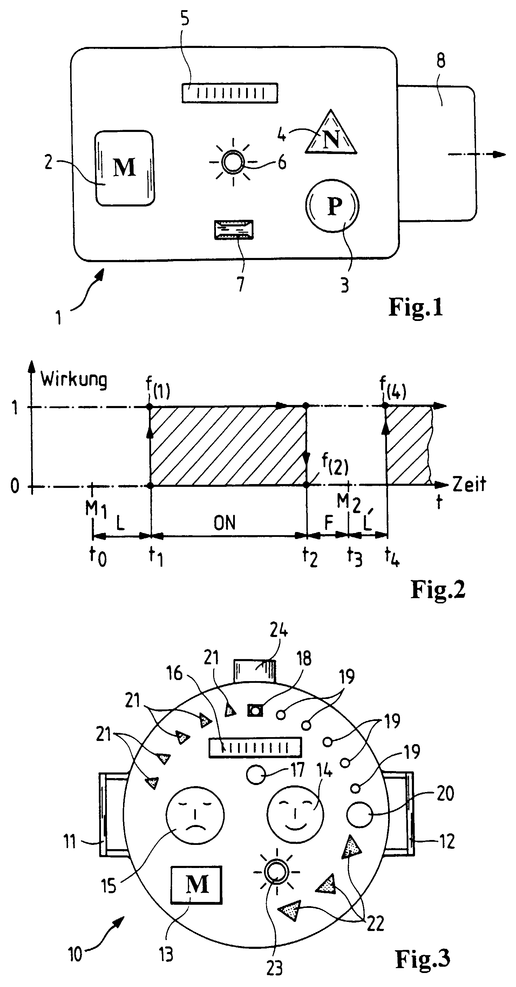

**Fig. 1** zeigt das am Körper des Patienten tragbare Registriergerät 1, welches etwa die Größe einer Armbanduhr oder eines Taschenrechners hat. Dieses Gerät weist auf seiner in der Zeichnung dargestellten Frontseite drei Tasten 2, 3 und 4 auf, die mit den Großbuchstaben M, N und P versehen sind. Darüber hinaus ist ein Display 5 vorgesehen, welches die seit Medikamenteneinnahme laufende Zeit und/oder die Uhrzeit anzeigt, die jeweils über eine Taste 7 gewählt werden kann. Schließlich ist ein herausnehmbarer Chip 8 dargestellt, der als Datenträger die aufgezeichneten Werte enthält. Das Gerät wird wie folgt gehandhabt: Der Patient, der das Gerät am Körper oder bei sich trägt, nimmt zunächst das vom Arzt verordnete Medikament ein und betätigt gleichzeitig die Taste M. Dadurch wird eine Stoppuhr in Gang gesetzt, d.h. die Zeit beginnt bei Null an zu laufen und wird optional am Display 5 angezeigt. Wenn nun der Patient nach Einnahme des Medikaments eine positive Wirkung verspürt, drückt er die P-Taste 3. Dieses Drücken der Taste quittiert das Gerät mit einem akustischen (Piepen) oder einem optischen Signal eines Lämpchens 6, so daß der Patient eine Bestätigung für seine Eingabe erhält. Verspürt der Patient nach geraumer Zeit eine nachlassende Wirkung, so drückt er die N-Taste 4. Danach nimmt er gemäß vom Arzt vorgegebener Anweisung zum zweiten Mal sein Medikament ein und drückt dabei wiederum die M-Taste 2, wodurch der Zeitpunkt gespeichert wird. Anschließend wiederholt sich der oben beschriebene Vorgang, d.h. Drücken der P-Taste bei Feststellen einer positiven Wirkung und Drücken der N-Taste bei nachlassender Wirkung. Dies erfolgt wohlgemerkt jeweils nach dem subjektiven Empfinden des Patienten.

Das Ergebnis eines solchen einfachen Eingabezyklus durch den Patienten ist in dem Diagramm gemäß **Fig. 2** dargestellt. Dort ist über der Zeitachse t die positive Wirkung des Medikaments auf der Ordinate aufgetragen, und zwar nur als ein positiver Wert der Größe 1. Der Beginn des Meßzyklus bei Medikamenteneinnahme ist durch den Punkt $M_1$ auf der Abszisse, d.h. der Zeitachse, markiert. Dort ist also die Medikamentenwirkung gleich 0. Nach einer geraumen Zeit, der sogenannten Latenzzeit L, verspürt der Patient erstmals eine positive Wirkung, er drückt die Taste P, wodurch der Wert f(1)=1 zum Zeitpunkt $t_1$ registriert wird. Nach einer weiteren Zeitspanne zum Zeitpunkt $t_2$ verspürt der Patient eine nachlassende oder gänzlich verschwindende Wirkung, drückt die Taste N, woduch der Wert f(2)=0 registriert wird. Der nächste Zeitpunkt auf der Zeitachse ist $M_2$, d.h. der Zeitpunkt $t_3$, zu dem das Medikament zum zweiten Mal eingenommen wird. $M_2$ wird durch Drücken der M-Taste registriert - wie man sieht, ist zu diesem Zeitpunkt $t_3$ die Wirkung des Medikaments noch Null, so daß sich eine sogenannte Fehlzeit F ergibt, d.h. eine Zeitspanne $t = t_3 - t_2$ ohne Medikamentenwirkung. Nach der zweiten Medikamenteneinnahme $M_2$ verstreicht wiederum eine Latenzzeit L', d.h. die Zeitdifferenz bis zum Zeitpunkt $t_4$, zu dem der Patient wiederum eine positive Wirkung verspürt und die P-Taste 3 drückt. Diese zweite Latenzzeit L' kann von der ersten L verschieden sein. Der so begonnene Meßzyklus kann beliebig durch wiederholte Einnahme der Medikamente zu den weiteren Zeitpunkten M3, M4, . . . $M_i$ mit entsprechender Registrierung der Wirkung fortgesetzt werden. Das Ergebnis ist als Funktion nach dem Muster in Figur 2 gespeichert und kann als Datenträger, z.B. auf dem Chip, entnommen werden. Es wäre auch möglich, die gespeicherte Funktion über einen PC-Adapter auf einem PC direkt zu analysieren, z.B. durch Vergleich der Wirkungsprofile an verschiedenen Tagen oder auszudrucken. Diese Daten, d.h. die Wirkfunktion dient dem Arzt als Hilfe, eine korrigierte bzw. optimale Medikamenteneinstellung für den Patienten vorzunehmen, und zwar mit dem Ziel, die oben erwähnten Fehlzeiten F und daran anschließende wiederholte Latenzzeiten L', d.h. die Zeitspannen ohne Medikamentenwirkung zu eliminieren.

**Fig. 3** zeigt ein weiteres Ausführungsbeispiel der Erfindung, und zwar das Registriergerät 10 in Form einer Armbanduhr mit Befestigungsösen 11 und 12 für ein nicht dargestelltes Armband. Das Gerät 10, welches also im wesentlichen in seiner Draufsicht kreisförmig ausgebildet ist, weist zunächst die M-Taste 13 sowie die weiteren Tasten 14 und 15 zur Eingabe einer positiven und einer negativen Wirkung auf, wobei die Taste 14 ein lachendes Gesicht und die Taste 15 ein trauriges Gesicht zeigt. Ferner ist ein Display 16 zur wahlweisen Anzeige einer laufenden (gestoppten) Zeit und der Uhrzeit vorgesehen, wobei der entsprechende Modus durch die Taste 17 gewählt werden kann. Das Gerät 10 weist ferner fünf optische Einrichtungen 19 (Leuchtdioden) auf, die einen jeweils registrierten Wert repräsentieren. Entsprechend sind auf der linken Seite des kreisförmigen Gerätes 10 fünf optische Einrichtungen 21 (Leuchtdioden) zur Anzeige der jeweils registrierten negativen Wirkung vorgesehen. Zwischen diesen beiden 5-er-Gruppen 19 und 21 ist eine Leuchteinrichtung 18 für die Nullstellung, d.h. keine Wirkung vorgesehen. Ferner weist das Gerät 10 eine vergrößerte Leuchteinrichtung 20 auf, die ein Optimum für das Befinden des Patienten signalisieren soll. Schließlich sind drei weitere Leuchteinrichtungen 22 für eine sogenannte überschießende Wirkung des Medikaments vorgesehen. Die vorgenannten Gruppen von Leuchteinrichtungen sind geometrisch unterschiedlich ausgebildet und leuchten in unterschiedlichen Farben auf, z.B. die Gruppe 19 und 20 grün, die Gruppe 21 blau und die Gruppe 22 gelb. Schließlich ist im Bereich des "Ziffernblattes" des Gerätes 10 eine akustische oder optische Signaleinrichtung 23 vorgesehen, die zum vorprogammierten Zeitpunkt der Medikamenteneinnahme (M1, M2, M3 . . .) akustische oder optische Signale abgibt, um den Patienten an die Medikamenteneinnahme zu erinnern. Das Signal kann auch durch Vibration erzeugt werden. Letztendlich weist das Gerät eine Anschlußstelle 24 für einen nicht dargestellten Adapter auf, über welchen die gespeicherten Daten auf einen PC übertragen und auf dessen Bildschirm sichtbar gemacht werden können.

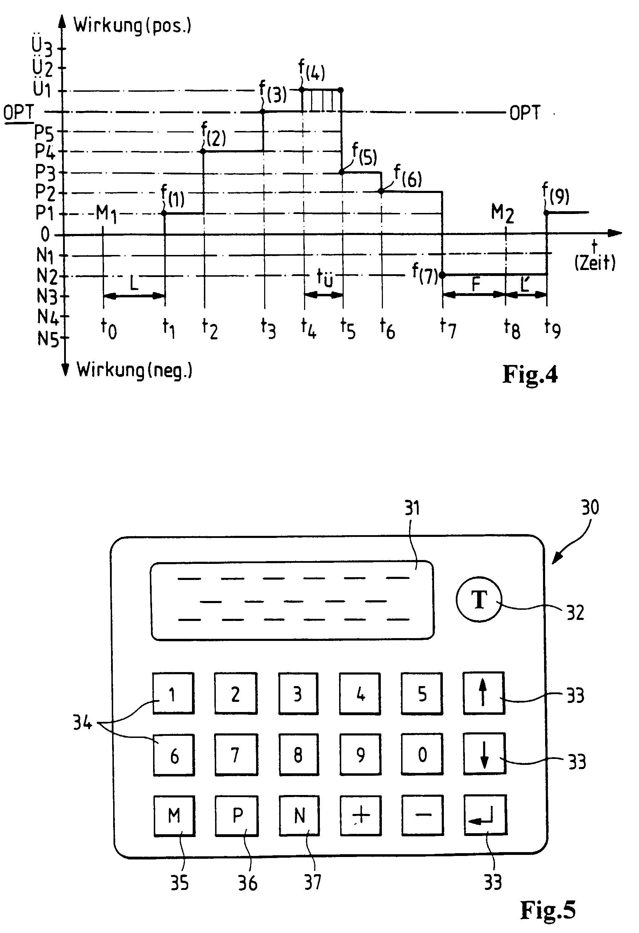

Die mit dem Gerät 10 gemäß Figur 3 aufgezeichnete und gespeicherte Funktion ist in dem Diagramm von **Fig. 4** dargestellt. Über der Zeitachse t ist sowohl die positive als auch die negative Wirkung nach Medikamenteneinnahme aufgetragen, wobei für die positive Wirkung fünf Werte P1 bis P5 und für die negative Wirkung fünf Werte N1 bis N5 vorgesehen sind. Oberhalb des Wertes P5 ist ein Wert OPT, repräsentativ für das Optimum, d.h. das optimale Empfinden des Patienten vorgesehen und darüber drei weitere Werte für eine überschießende Wirkung des Medikaments Ü1, Ü2 und Ü3. Das dargestellte Diagramm stellt den Wirkungsverlauf zwischen der Medikamenteneinnahme zum Zeitpunkt M1 und M2 dar und wird durch Betätigen der Tasten 13 (M), 14 für positive Wirkung und 15 für negative Wirkung registriert. Der Meßzyklus beginnt zum Zeitpunkt $t_0$, zu dem der Patient bei der ersten Medikamenteneinnahme die Taste M bzw. 13 drückt. Nach Verstreichen der Latenzzeit L verspürt er eine erste positive Wirkung und drückt zum Zeitpunkt $t_1$ einmal die Taste 14, wodurch der Wert f (1), entsprechend dem Wert P1 auf der positiven Ordinate registriert wird. Zum Zeitpunkt $t_2$ verspürt der Patient eine ansteigende positive Wirkung, die er, entsprechend seinem subjektiven Empfinden mit dem Wert P4 bewertet - dafür muß er die Taste 14 dreimal hintereinander drücken, wodurch sich ein Sprung von P1 auf P4 zum Wert f(2) ergibt. Mit dem Registrieren des Wertes P1 leuchtet die erste Leuchteinrichtung 19 (grün), mit dem Registrieren des Wertes P4 drei weitere Leuchteinrichtungen 19, also insgesamt vier Leuchteinrichtungen 19 auf. Zum Zeitpunkt $t_3$ verspürt der Patient eine optimale Wirkung, er drückt noch einmal die Taste 14, wodurch er den Wert P5 erreicht und noch einmal die Taste 14, wodurch der Wert OPT, d.h. das Optimum, entsprechend f(3) erreicht wird - gleichzeitig leuchtet eine vergrößerte Leuchteinrichtung 20 (grün) auf. Der Patient sieht somit, daß jetzt das Optimum der Medikamentenwirkung registriert ist. Zum Zeitpunkt $t_4$ empfindet er eine überschießende Wirkung des Medikaments - er drückt daher nochmals die Taste 14, wodurch der Wert f(4), entsprechend Ü1 auf der Ordinate registriert wird. Mit Erreichen des Wertes Ü1, d.h. bei Registrieren einer überschießenden Wirkung, leuchtet die zusätzliche Leuchteinrichtung 22 (gelb) auf, die somit den Bereich der überschießenden Wirkung optisch signalisiert. Empfindet der Patient ein Nachlassen dieser überschießenden Wirkung, kann er die Taste 15 drücken, wodurch bei einmaligem Drücken wieder der Wert OPT (Optimum) erreicht wird. Bei weiter nachlassender Wirkung kann der Patient wiederum die Taste 15 drücken: Bei einmaligem Drücken wird jeweils eine Einheit subtrahiert. Im dargestellten Fall gelangt er durch viermaliges Drücken der Taste 15 zum Zeitpunkt $t_5$ auf den Wert P3 entsprechend f(5) und durch weiteres Drücken zum Zeitpunkt $t_6$ auf den Wert P2 entsprechend f(6). Zum Zeitpunkt $t_7$ empfindet der Patient eine stark nachlassende Wirkung, die bis in den negativen Bereich absinkt. Er drückt daher die Taste 15 viermal und gelangt zum negativen Wert f(7) der N2 entspricht. Gleichzeitig leuchten jetzt zwei Leuchteinrichtungen 21 (blau) auf; entsprechend der negativen Werteskala N1 bis N5 sind fünf Leuchtdioden 21 (blau) vorgesehen. Der nächste Zeitpunkt $t_8$ entspricht dem vorgesehenen Zeitpunkt M2 für die zweite Medikamenteneinnahme. In diesem Falle besteht also noch eine negative Medikamentenwirkung, die noch bis zum Zeitpunkt $t_9$ anhält: erst dann verspürt der Patient wieder eine positive Wirkung, er drückt dreimal auf die Taste 14 mit dem lachenden Gesicht und gelangt zu dem Wert f(9), der dem Wert P1 auf der Ordinate entspricht. Die Zeitspanne zwischen $t_8$ und $t_7$ entspricht der Fehlzeit F ohne positive Medikamentenwirkung, und die Zeitspanne zwischen $t_9$ und $t_8$ ist die zweite Latenzzeit L' nach Einnahme des zweiten Medikaments. Danach wird der Meßzyklus wie vorher beschrieben fortgesetzt. Die Leuchtdiodengruppen 19 bis 22 können jeweils unterschiedliche Farben (wie oben angedeutet) aufweisen oder in gleicher Farbe, aber unterschiedlicher Helligkeit oder Umrißform aufleuchten (bei Farbenblindheit des Patienten). Dieses Diagramm, welches auf einem Datenträger gespeichert oder über einen Adapter auf einen PC übertragen werden kann, dient dem Arzt als Grundlage für eine genauere Medikamenteneinstellung, d.h. einerseits hinsichtlich der Wahl der Zeitpunkte M1, M2, M3 usw. als auch hinsichtlich der Dosierung des Medikaments. Letzteres ergibt sich beispielsweise bei Auftreten einer überschießenden Wirkung - hier bis zum Wert Ü1 über den Zeitraum $t_ü$ - da in diesem Falle die Dosierung des Medikaments zu stark war.

**Fig. 5** zeigt schließlich eine weitere Ausführung der Erfindung, und zwar als Gerät 30, welches ein vergrößertes Display bzw. einen kleinen Bildschirm 31 sowie eine Tastatur 33, 34 sowie die bereits beschriebenen Tasten mit den Großbuchstaben M, P und N entsprechend den Bezugsziffern 35, 36, 37 aufweist. Darüber hinaus ist ein Zeitumschalter 32 mit dem Großbuchstaben T vorgesehen, der ein wahlweises Umschalten auf Stoppuhr oder Uhrzeit erlaubt. Den Tasten 34 mit den Ziffern 1, 2, 3 bis 0 entsprechen Nebenwirkungen, z.B. Kopfschmerzen, Übelkeit, Fieber oder Herzrasen. Der Patient ist somit in der Lage, die nach Einnahme des Medikaments auftretende Nebenwirkung durch Bedienen der Tastatur am Bildschirm aufzurufen und mit dem Zeitpunkt des Auftretens registrieren zu lassen. Neben der Eingabe von Nebenwirkungen ist es auch möglich, spezifische Befindlichkeiten, die durch das Medikament beeinflußt werden sollen, z.B. den Tremor (Zitterbewegung von Körperteilen), die Muskelbeweglichkeit, Angst oder Erregtheit am Gerät auszuwählen, zu bewerten und den Zeitpunkt des Auftretens zu registrieren. Hierdurch können parallel zueinander unterschiedliche Wirkungsprofile gespeichert werden. Schließlich erlaubt das Programm dieses Gerätes 30, die Wirkung bei Einnahme von Medikamentenkombinationen, also mehreren Medikamenten gleichzeitig, zu registrieren.

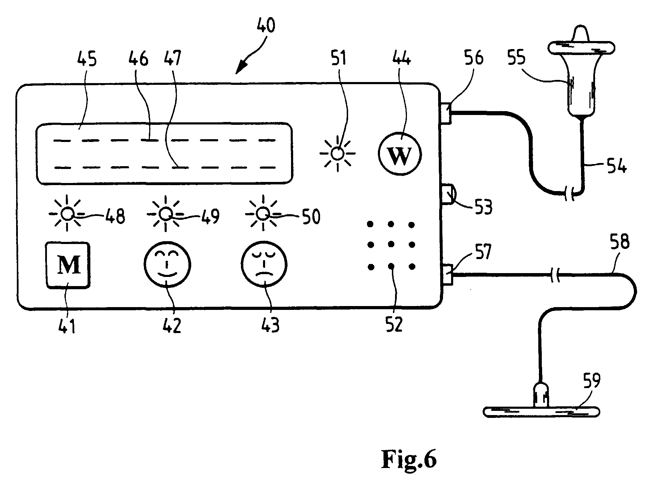

**Fig. 6** zeigt eine weitere Ausführungsform des erfindungsgmäßen Gerätes mit erweitertem Modus für die überlagerte Registrierng von zusätzlichen Ereignissen und Symptomen der Erkrankung sowie deren Bewertung. Das Gerät 40 weist - ähnlich wie das Gerät 1 und 10 - drei Tasten auf, die den Tasten M, N und P bzw. 13, 14 und 15 entsprechen, d.h. die Taste 41 mit dem Buchstaben M ist für die Eingabe des Zeitpunktes der Medikamenteneinnahme bestimmt, die Taste 42 mit lachendem Gesicht für die Eingabe einer positiven und die Taste 43 mit dem traurigen Gesicht für die Eingabe einer negativen Wirkung bzw. Bewertung bestimmt. Ferner ist eine Taste 44 mit dem Buchstaben W vorgesehen, über welche auf einem zweizeiligen Display 45 bestimmte Ereignisse und Symptome von bestimmten Krankheiten aufgerufen und angezeigt werden können. Z.B. können in der oberen Zeile 46 des Displays 45 verschiedene Oberbegriffe wie Zittern, Beweglichkeit, Kopfschmerzen, Übelkeit oder Angst durch Betätigen der Taste 44 (W) aufgerufen und angezeigt werden. In der unteren Zeile 47 erscheint dann eine Bewertungsmöglichkeit für das entsprechende Ereignis. Durch Betätigen der Taste 42 oder 43 kann dann eine Bewertung in verschiedenen Stufen erfolgen, z.B. bei "Kopfschmerzen

| | |
|---|---|
| keine Kopfschmerzen | (0) |
| schwache Kopfschmerzen | (1) |
| leichte Kopfschmerzen | (2) |
| mittelstarke Kopfschmerzen | (3) |
| starke Kopfschmerzen | (4) |
| extrem starke Kopfschmerzen | (5) |

Eine solche Bewertung erfolgt nicht auf Abfrage, sondern dann, wenn der Patient sie verspürt. Das Ereignis und seine Bewertung wird somit der Aufzeichnung der oben erwähnten Medikamentenwirkung gemäß **Fig. 2** und **Fig. 4** überlagert. Damit sind für den Arzt zusätzliche Informationen gegeben, die ihm eine genaue Beurteilung der Medikamentenwirkung erlauben. Für die optische Bestätigung der einzelnen Eingaben über die Tasten 41, 42, 43 und 44 sind diesen jeweils farbige Lämpchen 48, 49, 50 und 51 zugeordnet, die bei erfolgter Eingabe jeweils aufleuchten, und zwar gelb (48), grün (49), rot (50) und blau (51). Ferner weist das Gerät 40 einen kleinen Lautsprecher 52 auf, über den bestimmte Eingaben sprachlich hörbar bestätigt werden, z.B. "sagt" das Gerät 40 über die Sprachausgabe folgende Sätzde oder Worte: "Medikamenteneinnahme registriert" oder "Medikamentenwirkung jetzt: leichte Besserung". An dem Gerät 40 ist ferner ein Druckschalter 53 angeordnet, mit dem der Lautsprecher 52 ein- und ausgeschaltet werden kann.

Über ein Kabel 54 ist an das Gerät 40 ein durch den Patienten bedienbarer Taster 55 mittels Anschlußbuchse 56 anschließbar, über welchen zusätzlich zu den Tasten 42 und 43 Eingaben vorgenommen werden können. Dieser Taster 55 stellt quasi eine Fernbedienung des Gerätes 40 in bestimmten Fällen dar: z.B. können durch Drücken des Tasters 55 zusätzliche, plötzlich auftretende Ereignisse mit ihrer Uhrzeit aufgenommen werden, wobei der Patient das Gerät 40, welches er bei sich trägt, nicht aus der Tasche nehmen muß. Dies ist insbesondere bei Parkinson-Patienten bei Auftreten der sogenannten Off-Phase von Vorteil, weil der Patient dann in seiner Bewegungsfähigkeit stark eingeschränkt ist. Auch bei Angstattacken oder anderen krisenhaften, plötzlich auftretenden Befindlichkeitsänderungen, z.B. Absencen bei Epileptikern, erlaubt die sofortige Betätigung des Tasters 55 die sofortige Registrierung dieses Ereignisses. Der Taster 55 kann aber auch alternativ zu den Eingabetasten 42 und 43 eingesetzt werden, also zur Eingabe einer positiven oder negativen Medikamentenwirkung. Hierfür kann ein für den Patienten leicht verständlicher Code festgelegt werden: Z. B. würde ein kurzes Drücken des Tasters 55 bedeuten, daß keine Medikamentenwirkung vorliegt, und ein langes Drücken des Tasters 55, daß eine positive Wirkung eingetreten ist. Diese Ferneingabe über den Taster 55 kann dann akustisch über die Sprachausgabe 52 des Gerätes 40 bestätigt werden, so daß der Patient weiß, was registriert wird. Alternativ ist auch eine Bestätigung durch Vibration möglich.

Schließlich ist an dem Gerät 40 noch ein Anschluß 57 für diverse Meßfühler oder Meßgeräte vorgesehen, über die physikalisch meßbare Patientendaten erfaßt und registriert werden. Hier ist beispielhaft und schematisch ein über ein Kabel 58 angeschlossener Meßfühler 59 zur Messung der Herzfrequenz des Patienten dargestellt. Alternativ oder zusätzlich können weitere Meßfühler, z.B. zur Messung des Blutdruckes, des Blutsauerstoff- bzw. -zuckergehaltes, des Tremors, des Muskeltonus (Muskelanspannung) sowie der Hauttemperatur oder -feuchtigkeit angeschlossen werden. Diese objektiv feststellbaren und meßbaren Werte können ohne das Zutun des Patienten automatisch gemessen und aufgezeichnet und auf dem oben erwähnten Datenträger gespeichert und entnommen werden. Insofern ist es möglich, durch dieses Kombinationsgerät 40 sowohl die subjektive Befindlichkeit nach der Medikamenteneinnahme als auch die objektiv festgestellten physiologischen Werte parallel zu erfassen und zu speichern. Dies stellt eine wesentliche Therapiehilfe für den Arzt und eine Verbesserung der Medikamenteneinstellung für den Patienten dar.

Nicht dargestellt in der Zeichnung ist eine Kombination des oben beschriebenen Gerätes mit einem Medikamentenspender, der die für die Einnahme verordneten Medikamente für einen bestimmten Zeitraum in entsprechender Menge enthält. Zu den vorgegebenen Zeitpunkten $M_1$, $M_2$, $M_3$ . . ., die im Gerät gespeichert sind, öffnet der Medikamentenspender und teilt die verordnete Medikamentendosis zu, d.h. der Patient kann das Medikament dann entnehmen. Gleichzeitig kann die oben beschriebene Signal- oder Weckeinrichtung in Funktion treten und den Patienten damit an die Medikamenteneinnahme erinnern. Dabei können die Medikamente, die zu diesem Zeitpunkt eingenommen werden sollen, angezeigt werden, z.B.:

" 1/2 Tablette Madopar 125 T ".

Wie bereits oben erwähnt, ist das erfindungsgemäße Gerät nicht nur während der medikamentösen Behandlung eines Patienten durch einen Arzt einsetzbar, sondern beispielsweise auch in der Pharmaforschung, wenn neue Wirksubstanzen, Heilmittel, Arzneimittel oder dergleichen im Hinblick auf ihre Wirkung auf den menschlichen Körper untersucht und ausgewertet werden sollen.

**Patentansprüche**

1. Tragbares Gerät (1, 10, 30, 40) nach Art eines Taschencomputers zur Registrierung. Anzeige und Speicherung von subjektiven Befindlichkeitswerten, die durch einen Patienten selbst mittels Betätigungseinrichtungen eingebbar sind, **dadurch gekennzeichnet**, daß die Betätigungseinrichtungen

   a) aus einer ersten Taste (M, 2, 13, 35, 41) zur Registrierung des Zeitpunktes ($t_0$) der Medikamenteneinnahme (M1, M2, M3 ...).

   b) aus einer zweiten Taste (P, 3, 14, 36, 42) zur Registrierung einer positiven Wirkung und des Zeitpunktes ($t_1$) sowie

   c) aus einer dritten Taste (N, 4, 15, 37, 43) zur Registrierung einer negativen Wirkung und des Zeitpunktes ($t_2$) bestehen,
   wobei die Werte gemäß a), b) und/oder c) beliebig oft und jeweils bei Auftreten einer Wirkungsänderung eingebbar sind,
   und daß die eingegebenen Werte gemäß a), b) und c) zu einer Funktion der Medikamentenwirkung über der Zeit auf einem Datenträger (8) aufgezeichnet und darstellbar sind.

2. Gerät nach Anspruch 1, **dadurch gekennzeichnet**, daß der Datenträger (8) entnehmbar ist.

3. Gerät nach Anspruch 1, **dadurch gekennzeichnet**, daß das Gerät (10) eine Schnittstelle (24) für einen PC-Adapter aufweist, über welchen die gespeicherten Daten auf einen PC übertragbar sind.

**4.** Gerät nach Anspruch 1, 2 oder 3, **dadurch gekennzeichnet**, daß das Gerät (1, 10, 30, 40) ein Display (5, 16, 31, 45) zur Anzeige von Informationen, z.B. der laufenden Zeit (Stoppuhr) ab Medikamenteneinnahme und/oder der Tageszeit und den Grad der Befindlichkeit aufweist.

**5.** Gerät nach einem der Ansprüche 1 bis 4, **dadurch gekennzeichnet**, daß das Gerät (1, 10, 40) eine akustische, taktile (durch Vibration) und/oder visuelle Bestätigungseinrichtung (6; 17, 18, 19, 20, 21, 22, 23; 48, 49, 50, 51, 52) für jede Eingabe aufweist.

**6.** Gerät nach Anspruch 5, **dadurch gekennzeichnet**, daß die akustische Bestätigungseinrichtung ein Sprachmodul mit einem Lautsprecher (52) umfaßt, welches die jeweilige Eingabe sprachlich hörbar bestätigt.

**7.** Gerät nach einem der vorhergehenden Ansprüche, **dadurch gekennzeichnet**, daß das Gerät (40) zusätzlich zu den Tasten (42, 43) einen über Kabel (54) anschließbaren Taster (55) aufweist, über welchen von Hand Informationssignale in das Gerät (40) eingebbar sind.

**8.** Gerät nach einem der vorhergehenden Ansprüche, **dadurch gekennzeichnet**, daß das Gerät (10, 30, 40) eine Signaleinrichtung (23, 31, 45, 52) aufweist, welche dem Patienten die vorgegebenen Zeitpunkte ($M_1$, $M_2$, $M_3$, ...) für die Medikamenteneinnahme akustisch oder visuell oder durch Vibration signalisiert.

**9.** Gerät nach einem der vorhergehenden Ansprüche, **dadurch gekennzeichnet**, daß das Gerät (10) optisch erkennbare Anzeigevorrichtungen (18, 19, 20, 21, 22) zur Sichtbarmachung der jeweils registrierten positiven (19), negativen (21) oder überschießenden (22) Werte oder des Optimums (20) aufweist.

**10.** Gerät nach einem der vorhergehenden Ansprüche, **dadurch gekennzeichnet**, daß das Gerät (10) die Form und Größe einer Armbanduhr aufweist und am Handgelenk des Patienten tragbar ist.

**11.** Gerät nach einem der Ansprüche 4 bis 10, **dadurch gekennzeichnet**, daß auf dem Display (45) zusätzlich auftretende Ereignisse und deren Bewertung anzeigbar sind, wobei die Ereignisse über eine zusätzliche Taste W (44) anzeigbar und deren Bewertungen über die Eingabetasten P (42) und N (43) eingebbar sind.

**12.** Gerät nach einem der Ansprüche 1 bis 10, **dadurch gekennzeichnet**, daß das Gerät (30) weitere gekennzeichnete Tasten (Tastatur 0, 1, 2, 3; M, P, N ...) zur Registrierung von im Gerät eingegebenen Nebenwirkungen bzw. spezifischen Befindlichkeiten, die der Patient empfindet, aufweist.

**13.** Gerät nach einem der Ansprüche 1 bis 12, **dadurch gekennzeichnet**, daß das Gerät (40) zusätzlich eine Meß- oder Fühleinrichtung (59) zum Feststellen und Registrieren von physikalisch meßbaren physiologischen Werten des Patienten aufweist.

**14.** Gerät nach einem der vorhergehenden Ansprüche, **dadurch gekennzeichnet**, daß mit dem Gerät (1, 10, 30, 40) ein Medikamentenspender verbunden ist, der die Medikamente enthält und diese zu den vorgegebenen Zeitpunkten ($M_1$, $M_2$, $M_3$, . . .) in entsprechender Dosis zuteilt.

## Claims

**1.** Portable equipment (1, 10, 30, 40) in the manner of a pocket computer for recording, displaying and storing values concerning a patient's subjective state of health, the values being input by a patient himself by means of operating arrangements, characterized in that the operating arrangements consist of

a) a first button (M, 2, 13, 35, 41) for recording the time ($t_0$) at which the medication was taken (M1, M2, M3, etc.),
b) a second button (P, 3, 14, 36, 42) for recording a positive effect and the time ($t_1$), and
c) a third button (N, 4, 15, 37, 43) for recording a negative effect and the time ($t_2$),
where the values according to a), b) and/or c) can be input as often as desired and each time a change in effect occurs,
and in that the input values according to a), b) and c) as a function of the effect of the medication with time are recorded on a recording medium (8) and can be reproduced.

**2.** Equipment according to Claim 1, characterized in that the recording medium (8) can be removed.

**3.** Equipment according to Claim 1, characterized in that the equipment (10) has an interface (24) for a PC adapter via which the stored data can be transferred to a PC.

**4.** Equipment according to Claim 1, 2 or 3, characterized in that the equipment (1, 10, 30, 40) has a display (5, 16, 31, 45) for displaying information, e.g. the time elapsing (stopwatch) from the time the medication was taken and/or the time of day and the state of health.

**5.** Equipment according to one of Claims 1 to 4, characterized in that the equipment (1, 10, 40) has an acoustic, tactile (by vibration) and/or visual confirmation arrangement (6; 17, 18, 19, 20, 21, 22, 23; 48, 49, 50, 51, 52) for each entry.

**6.** Equipment according to Claim 5, characterized in that the acoustic confirmation arrangement includes a speech module with a speaker (52) which provides an audible spoken confirmation of the respective entry.

**7.** Equipment according to one of the preceding claims, characterized in that the equipment (40) has, in addition to the buttons (42, 43), a key switch (55) which can be connected via cable (54) and by means of which information signals can be input to the equipment (40) by hand.

**8.** Equipment according to one of the preceding claims, characterized in that the equipment (10, 30, 40) has a signal arrangement (23, 31, 45, 52) which signals to the patient, either acoustically or visually, or by vibration, the preset times ($M_1$, $M_2$, $M_3$, etc.) at which the medication is to be taken.

**9.** Equipment according to one of the preceding claims, characterized in that the equipment (10) has optical display devices (18, 19, 20, 21, 22) for showing the respectively recorded positive (19), negative (21) or excess (22) values or the optimum (20).

**10.** Equipment according to one of the preceding claims, characterized in that the equipment (10) is the shape and size of a wristwatch and can be worn on the patient's wrist.

**11.** Equipment according to one of Claims 4 to 10, characterized in that additional events and their assessment can be displayed on the display (45), in which case the events can be displayed via an additional button W (44) and their assessments can be input via the input buttons P (42) and N (43).

**12.** Equipment according to one of Claims 1 to 10, characterized in that the equipment (30) has further labelled buttons (keyboard 0, 1, 2, 3; M, P, N, etc.) for recording side effects input to equipment or specific feelings the patient experiences.

**13.** Equipment according to one of Claims 1 to 12, characterized in that the equipment (40) has in addition a measurement or sensor arrangement (59) for detecting and recording physically measurable physiological values of the patient.

**14.** Equipment according to one of the preceding claims, characterized in that a medication dispenser connected to the equipment (1, 10, 30, 40) contains the medication and dispenses this at the preset times ($M_1$, $M_2$, $M_3$, etc.) in the appropriate dose.

**Revendications**

**1.** Appareil portable (1, 10, 30, 40) du type ordinateur de poche, pour l'enregistrement, l'indication et la mémorisation de valeurs subjectives relatives à l'état de santé ressenti, qui peuvent être saisies par un patient lui-même au moyen de dispositifs d'actionnement, **caractérisé** en ce que les dispositifs d'actionnement sont constitués par

a) une première touche (M, 2, 13, 35, 41) pour l'enregistrement de l'instant ($t_0$) de la prise de médicaments (M1, M2, M3, ...),
b) une seconde touche (P, 3, 14, 36, 42) pour l'enregistrement d'un effet positif et de l'instant ($t_1$), ainsi
c) qu'une troisième touche (N, 4, 15, 37, 43) pour l'enregistrement d'un effet négatif et de l'instant ($t_2$),
les valeurs selon a), b) et/ou c) pouvant être saisies aussi souvent que souhaité et respectivement à l'apparition d'une variation d'effet, et en ce que les valeurs saisies selon a), b) et c) peuvent être enregistrées sur un support de données (8) et représentées, sous la forme d'une fonction de l'effet des médicaments par rapport au temps.

**2.** Appareil selon la revendication 1, **caractérisé** en ce que le support de données (8) est amovible.

**3.** Appareil selon la revendication 1, **caractérisé** en ce que l'appareil (1) comporte une interface (24) pour un adaptateur d'ordinateur personnel, par l'intermédiaire duquel les données mémorisées peuvent être transférées à un ordinateur personnel.

**4.** Appareil selon la revendication 1, 2 ou 3, **caractérisé** en ce que l'appareil (1, 10, 30, 40) comporte un écran (5, 16, 31, 45) pour la visualisation d'informations, par exemple du temps qui s'écoule (chronomètre) à partir de la prise de médicaments, et/ou de l'heure du jour et du degré de l'état de santé ressenti.

**5.** Appareil selon l'une des revendications 1 à 4**, caractérisé** en ce que l'appareil (1, 10, 40) comporte un dispositif de confirmation (6; 17, 18, 19, 20, 21, 22, 23; 48, 49, 50, 51, 52) acoustique, tactile (par vibration) et/ou visuelle, pour chaque saisie.

**6.** Appareil selon la revendication 5, **caractérisé** en ce que le dispositif de confirmation acoustique comprend un module vocal avec un haut-parleur (52), qui confirme la saisie considérée de manière perceptible sur le plan vocal.

**7.** Appareil selon l'une des revendications précédentes, **caractérisé** en ce que l'appareil (40) comporte, en plus des touches (42, 43), un système à touche (55) pouvant être raccordé par l'intermédiaire d'un câble (54), et par lequel il est possible d'introduire, à la main, des signaux d'information dans l'appareil (40).

**8.** Appareil selon l'une des revendications précédentes, **caractérisé** en ce que l'appareil (10, 30, 40) comprend un dispositif de signalisation (23, 31, 45, 52) qui signale au patient, de manière acoustique ou visuelle ou par vibration, les instants prescrits (M1, M2, M3, ...) pour la prise de médicaments.

**9.** Appareil selon l'une des revendications précédentes, **caractérisé** en ce que l'appareil (10) comporte des dispositifs indicateurs (18, 19, 20, 21, 22) à perception optique, pour visualiser les valeurs positives (19), négatives (21) ou de dépassement ou la valeur optimum (20), respectivement enregistrées.

**10.** Appareil selon l'une des revendications précédentes, **caractérisé** en ce que l'appareil (10) présente la forme et la taille d'une montre-bracelet, et peut être porté au poignet du patient.

**11.** Appareil selon l'une des revendications 4 à 10, **caractérisé** en ce que sur l'écran (45) peuvent être visualisés des événements supplémentaires apparaissant et leur évaluation, les événements pouvant être visualisés par l'intermédiaire d'une touche supplémentaire W (44), et leurs évaluations pouvant être saisies par l'intermédiaire des touches de saisie P (42) et N (43).

**12.** Appareil selon l'une des revendications 1 à 10, **caractérisé** en ce que l'appareil (30) présente d'autres touches caractérisées (clavier 0, 1, 2, 3; M, P, N, ...) pour l'enregistrement d'effets secondaires introduits dans l'appareil ou d'états de santé ressentis, spécifiques, que perçoit le patient.

**13.** Appareil selon l'une des revendications 1 à 12, **caractérisé** en ce que l'appareil (40) comporte en supplément un système de mesure ou de sonde (59) pour relever et enregistrer des valeurs physiologiques du patient, pouvant être mesurées physiquement.

**14.** Appareil selon l'une des revendications précédentes, **caractérisé** en ce qu'à l'appareil (1, 10, 30, 40) est relié un distributeur de médicaments, qui contient les médicaments et qui délivre ceux-ci selon la dose appropriée aux instants (M1, M2, M3, ...) prescrits.

## Fig.1

## Fig.2

## Fig.3

Fig.4

Fig.5

**Fig.6**